# EUROPEAN PATENT APPLICATION

(11) **EP 4 670 652 A1**
(43) Date of publication of application: **31.12.2025**
(21) Application number: 23929933.2
(22) Date of filing: 07.11.2023
(51) Int. Cl.: A61B 17/221, A61B 17/22

(54) **MEDICAL DEVICE AND MEDICAL SYSTEM**

(30) Priority: 28.03.2023 CN 202310321106
(71) Applicant: Shanghai MicroPort Medical (Group) Co., Ltd., Shanghai 201203 (CN)
(72) Inventor: GENG, Kangkang, Shanghai 201203 (CN); ZHOU, Qi, Shanghai 201203 (CN); WU, Jintian, Shanghai 201203 (CN); HUANG, Haiyong, Shanghai 201203 (CN)
(74) Representative: Marks & Clerk LLP
(86) International application number: PCT/CN2023/130174
(87) International publication number: WO 2024/198363

(57) **Abstract**

The present invention provides a medical device and a medical system. The medical system includes a medical device, delivery device and an aspiration device. The medical device includes a supporting member and a mesh capture device. The mesh capture device is mounted over an outer circumference of the supporting member at a distal end thereof, and a plurality of capture units of the mesh capture device are sequentially arranged at an interval along an axial direction of the supporting member. In the plurality of capture units, a first capture unit is farther away from a proximal end of the supporting member than second capture units. The first capture unit has a first capture mesh outwardly folded along its entire circumference and curved proximally, and the second capture unit has a second capture mesh radially projecting outwardly along part of its circumference. The second capture meshes of adjacent second capture units form a capture mesh that is along the entire circumference of the supporting member. With this arrangement, both good compliance and desirable supporting performance can be obtained, and effective removal of old and strongly adherent occlusive objects from lumens can be achieved without escape of such occlusive objects.

## Description

### TECHNICAL FIELD

The present invention relates to the field of medical equipment, and particularly to a medical device and a medical system.

### BACKGROUND

An embolism refers to the phenomenon where an insoluble abnormal material enters the circulating blood, flows with the blood, and subsequently blocks the lumen of a blood vessel. A pulmonary embolism (PE) is a blockage of a pulmonary artery or a branch thereof by a detached blood clot or other material, which impairs pulmonary circulation. PEs are characterized by high incidence, high mortality, high recurrence, frequent missed diagnosis, etc. After the pulmonary artery or branch thereof is blocked, the effects of mechanical blockage and neurohumoral factors would lead to increased pulmonary circulation resistance, higher pulmonary arterial pressure, and abnormal ventilation-perfusion ratio, which may in turn cause a series of changes including right heart failure, decreased systemic blood pressure, congestion and severe hypoxemia. In severe cases, pulmonary infarction, pulmonary collapse, impaired functions of the heart, brain, kidneys and other important organs, or even sudden death may occur.

Conventional treatment of thromboembolisms and/or PEs involves reducing and/or removing abnormal material mainly by, among others, pharmaceutical anticoagulation, surgery or minimally invasive intervention. Pharmaceutical anticoagulation is conservative and suitable for patients with mild symptoms. However, patients with severe symptoms, who have failed medication, have to undergo a necessary surgical procedure. Compared with traditional surgical treatment, minimally invasive intervention features minimal trauma and fast recovery and is therefore regarded as a very promising technique for the treatment of acute embolisms or PEs.

Minimally invasive intervention often employs approaches such as thrombolysis, rotational atherectomy, aspiration, basket, stent, or balloon thrombectomy to remove thrombus and other abnormal materials, thereby restoring blood flow within the vessel lumen. Among these approaches, stent or basket-based thrombectomy devices have been recognized by patients and surgeons and become an intensively studied topic of research in recent years because of excellent clinical outcomes. By establishing a sheath, a basket or other thrombectomy component may be advanced to a thrombus site inside a blood vessel. After that, it may be released so as to penetrate and capture the blood clot and then withdrawn therewith out of the body through the access sheath. However, due to a lack of radial strength, conventional mesh capture device tends to deform when compressed by a thrombus, and during withdrawal, the thrombus tends to fall off the mesh surface or escape through the openings therein, leading to low thrombus success and an increased risk of distal occlusion by disrupted thrombus. Moreover, the thrombus may fail to be carried away during withdrawal if it is hard thrombus. There are also other such medical devices, which exhibit sufficient radial strength but lack compliance due to the high stiffness. These devices may get stuck and therefore be locally piled up, particularly when being retracted into a catheter, due to a limited luminal space of the catheter. When this happens, not only smooth retraction into the catheter may not be attained, but the captured thrombus may escape into a distal blood vessel. There are also some conventional thrombectomy components in the form of distally-closed mesh cylinders, which are inadequately compliant and may collapse when used in a tortuous blood vessel, leading to an increased risk of escape or disruption of the captured thrombus. Moreover, due to a larger contact area with the blood vessel, the risk of blood vessel damage is also increased. Further, it would be difficult for such components to detach, capture and remove a thrombus that firmly adheres to a blood vessel wall. When used to remove a stone from a bile duct, ureter or the like, the conventional devices would present problems similar to those with thrombus removal.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY OF THE INVENTION

It is an object of the present invention to provide a medical device and a medical system, which overcome the problems with conventional devices, including low treatment efficiency and low treatment success, when they are used to treat occlusive objects such as thrombi and stones.

To this end, according to a first aspect of the present invention, there is provided a medical device used to remove an occlusive object from a target lumen, which comprises a supporting member and a mesh capture device. The mesh capture device is mounted over an outer circumference of the supporting member at a distal end thereof.

The mesh capture device comprises a plurality of capture units which are sequentially arranged at an interval along an axial direction of the supporting member, wherein the plurality of capture units include a first capture unit and a plurality of second capture units. The first capture unit is farther away from a proximal end of the supporting member than the second capture units and has a first capture mesh outwardly folded along its entire circumference and curved proximally. Each of the second capture units has a second capture mesh radially projecting outwardly along part of its circumference. The second capture meshes of adjacent second capture units form a capture mesh that is along the entire circumference of the supporting member.

Optionally, the second capture meshes may be formed by a mesh tube that is outwardly folded along a part of a circumference thereof and is curved proximally, or by a mesh tube that radially projects outwardly along a part of a circumference thereof.

Optionally, the plurality of capture units may further include a third capture unit, wherein the first capture unit is farther away from the proximal end of the supporting member than the third capture unit, and the third capture unit has a third capture mesh radially projecting outwardly along its entire circumference.

Optionally, the third capture mesh may be formed by a mesh tube, which radial projects outwardly along the entirety of a circumference thereof, and/or the third capture mesh is deflected proximally or distally.

Optionally, the plurality of capture units may be integrally connected to form a single structure; or the plurality of capture units are disconnected to form separate structures.

Optionally, in case of the plurality of capture units being integrally connected to form a single structure, the mesh capture device is formed from a single mesh tube; adjacent capture units are connected by a straight loading section; a most distal capture unit is proximally attached to the supporting member to form a distal joint; a most proximal capture units is proximally attached to the supporting member to form a proximal joint; and the straight loading section of the mesh capture device is located between the proximal and distal joints and is not attached to the supporting member.

Optionally, in case of the plurality of capture units being disconnected to form separate structures, the mesh capture device is formed from a plurality of mesh tubes, wherein each capture unit is fabricated from a corresponding mesh tube and comprises a straight loading section, wherein the straight loading section is fixedly sleeved over the outer circumference of the distal end of the supporting member.

Optionally, the second capture meshes may be formed by the mesh tube that is outwardly folded along a part of the circumference thereof and is curved proximally, wherein each of the first capture mesh and/or the second capture meshes comprises a leading end and a trailing end opposite to each other along an axis thereof, the leading end is proximally curved, the trailing end is curved toward the leading end, and a mesh opening density of the leading end is lower than a mesh opening density of the trailing end.

Optionally, the second capture meshes may be formed by the mesh tube that is outwardly folded along a part of the circumference thereof and is curved proximally, wherein each of the first capture mesh and/or the second capture meshes is outwardly curved at a trailing end thereof to form a non-invasive end, the non-invasive end is covered with a soft material.

Optionally, the second capture meshes may be formed by the mesh tube that radially projects outwardly along a part of the circumference thereof, wherein the second capture mesh is defined by a portion of at least one shape selected from a sphere, a disk, a cylinder, a bag and a basket.

Optionally, the third capture mesh may be defined by at least one shape selected from a sphere, a disk, a cylinder, a bag and a basket.

Optionally, the medical device may further comprise at least one of:
a radial dimension of at least one of the capture units after fully expanded being greater than or equal to a diameter of the target lumen;
a mesh opening density of the capture meshes of at least some of the capture units gradually decreasing from its center to its periphery;
a mesh opening density of the mesh capture device gradually decreasing in a distal to proximal direction;
a radial dimension of the mesh capture device gradually increasing or decreasing in a proximal to distal direction; and
a graft provided on the first capture mesh, the graft covers some of mesh openings in the first capture mesh.

Optionally, the supporting member may comprise a hollow core and a distal guide head, the distal guide head provided at a distal end of the hollow core, the hollow core comprising a stiff section and an elastic section arranged along an axial direction from a proximal end to a distal end, wherein the mesh capture device is disposed on the elastic section.

To achieve the above end, according to a second aspect of the present invention, there is provided a medical system comprising the medical device as defined above; a delivery device for loading and delivering the medical device into a target lumen; and an aspiration device configured for connection with the delivery device and for application of a suction force through the delivery device for sucking an occlusive object inside the target lumen.

As described above, the present invention provides a medical device used to remove an occlusive object from a target lumen, which comprises a supporting member and a mesh capture device. The mesh capture device is mounted over an outer circumference of the supporting member at a distal end thereof. The mesh capture device comprises a plurality of capture units, which are sequentially arranged at an interval along an axial direction of the supporting member and include a first capture unit and a plurality of second capture units. The first capture unit is farther away from a proximal end of the supporting member than the second capture units and has a first capture mesh outwardly folded along its entire circumference and curved proximally. The second capture unit has a second capture mesh radially projecting outwardly along a part of its circumference. The second capture meshes of adjacent second capture units form a capture mesh that is along the entire circumference of the supporting member. In the context of thrombus removal from a blood vessel, as an example.

Firstly, the plurality of capture units sequentially arranged can segmentally remove the thrombus from the body of a patient, which has high thrombectomy efficiency and good thrombectomy performance. In particular, the capture units are meshes with sufficient radial strength and are therefore highly resistant to deformation due to compression by thrombus. Thus, they can better scrape off, capture and transport old and strongly adherent thrombi with good thrombectomy performance. In addition, the mesh structure comprises sufficiently dense mesh openings for thrombus capture and trapping without the captured thrombus falling off from the capture meshes or escape from one or more mesh openings, resulting in high thrombus capture success.

Secondly, in the plurality of capture units, the second capture meshes of the second capture units project outwardly along a part of a circumference thereof. This structural design increases the compliance and adaptation of the entire device within tortuous blood vessels and blood vessels with a varying diameter and reduces the likeliness of the meshes experiencing excessive deformation or inward collapse within such blood vessels, making the medical device suitable for use in various lumens. It also reduces resistance to retraction of the device into the delivery device. In addition, the second capture meshes of two adjacent second capture units can form a circumferentially complete capture mesh, which can facilitate thrombus capture and transport. Considering the risk of falling off and escape of thrombus from the second capture meshes, the first capture unit is added to the most distal end of the medical device, which is not only capable of thrombus capture itself, but can also trap thrombus escaping from the proximal end, increasing thrombus capture success. As the first capture unit employs an outwardly folded and curved structural design, it has a small contact area with a blood vessel, which enables less damage to be caused to a wall of the blood vessel during scraping off of a thrombus therefrom and during retraction, it allows the device to be smoothly guided and retracted into the delivery device without falling off or escape of the captured thrombus. To conclude, the combined use of the first capture unit and the multiple second capture units enable not only removal of the thrombus segmentally from a patient's body with higher thrombectomy efficiency but also both good overall compliance and sufficient support of the device, which can reduce possible damage to a blood vessel and resistance to retraction. In particular, effective removal of old and strongly adherent thrombi from blood vessels can be obtained, and escape of thrombus can be effectively blocked, resulting in higher thrombus capture success.

As the medical system of the present invention is based on the same inventive concept as the medical device of the present invention and thus has all the advantages of the medical device, the advantages thereof are not repeated herein.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a front view of a medical system according to a first embodiment of the present invention;
Fig. 2 shows a schematic perspective view of a medical device in a fully expanded configuration according to the first embodiment of the present invention;
Fig. 3 shows a front view of the medical device in the fully expanded configuration according to the first embodiment of the present invention;
Fig. 4a is a schematic partial view of the medical device in the fully expanded configuration according to the first embodiment of the present invention, showing adjacent second capture units;
Fig. 4b is a side view of the medical device in the fully expanded configuration according to the first embodiment of the present invention, which is a left-to-right view or right-to-left view of the front view of Fig. 4a;
Fig. 5 is a schematic perspective view of a distalmost first capture unit after fully expanded according to the first embodiment of the present invention;
Fig. 6a is a schematic perspective view of a second capture unit after fully expanded according to the first embodiment of the present invention;
Fig. 6b is a side view of the second capture unit of Fig. 6a;
Fig. 7a schematically illustrates a guide wire being advanced to a target lesion site during a surgical procedure according to the first embodiment of the present invention;
Fig. 7b schematically illustrates a sheath and a delivery catheter being successively advanced over the guide wire to a thrombus site during the surgical procedure according to the first embodiment of the present invention;
Figs. 7c to 7d schematically illustrate a plurality of capture units being released as a result of retracting the delivery catheter during the surgical procedure according to the first embodiment of the present invention;
Fig. 7e schematically illustrates the capture units having all been completely released into a fully expanded configuration where they penetrate into the thrombus during the surgical procedure according to the first embodiment of the present invention;
Fig. 7f schematically illustrates the medical device being withdrawn to transport and remove the thrombus during the surgical procedure according to the first embodiment of the present invention;
Fig. 8 is a front view of a first capture unit with a graft according to a second embodiment of the present invention;
Fig. 9a is a front view of a first capture unit containing a radiopaque element according to a third embodiment of the present invention;
Fig. 9b is a schematic partial view of the first capture unit of Fig. 9a;
Fig. 10a is a schematic diagram showing a first capture unit with a larger curvature angle according to a fourth embodiment of the present invention;
Fig. 10b is a schematic diagram showing a second capture unit with a larger curvature angle according to the fourth embodiment of the present invention;
Fig. 11a is a schematic diagram showing another example of the first capture unit according to the fourth embodiment of the present invention, in which a first capture mesh has a polygonal mesh at its trailing end and an arcuate mesh at its leading end;
Fig. 11b is a schematic diagram showing another example of the second capture unit according to the fourth embodiment of the present invention, in which a second capture mesh has a polygonal mesh at its trailing end and an arcuate mesh at its leading end;
Fig. 12a shows yet another example of the first capture unit according to the fourth embodiment of the present invention, in which a first capture mesh is curved at its trailing end;
Fig. 12b is a schematic partial view of the first capture unit of Fig. 12a;
Fig. 12c shows yet another example of the second capture unit according to the fourth embodiment of the present invention, in which a second capture mesh is curved at its trailing end;
Fig. 13a shows a further example of the first capture unit according to the fourth embodiment of the present invention, in which a first capture mesh has polygonal meshes respectively at its leading and trailing ends and an arcuate transition mesh connecting the two polygonal meshes;
Fig. 13b shows a further example of the second capture unit according to the fourth embodiment of the present invention, in which a second capture mesh has polygonal meshes respectively at its leading and trailing ends and an arcuate transition mesh connecting the two polygonal meshes;
Fig. 14 is a front view of a medical device according to a fifth embodiment of the present invention, in which a mesh capture device, when fully expanded, has a radial dimension gradually decreasing from a distal end to a proximal end;
Fig. 15a shows an example of a medical device according to a sixth embodiment of the present invention, in which capture units are connected together;
Fig. 15b shows another example of the medical device according to the sixth embodiment of the present invention, in which capture units show a mesh opening density gradually decreasing from the distal end to the proximal end;
Fig. 15c shows yet another example of the medical device according to the sixth embodiment of the present invention, in which a basket-shaped third capture unit is added.

### List of Reference Numerals

100-medical device; 110-supporting member; 111-distal guide head; 130 mesh capture device; 131-capture unit; 131a-straight loading section; 131b-trailing end; 131c-leading end; 131d-proximal end; 131 e-mesh opening; 131f-mesh strut; 131g-non-invasive end; 131h-soft material; 1311-first capture unit; 1311a-first capture mesh; 1312-second capture unit; 1312a-second capture mesh; 1313-third capture unit; 1313a-third capture mesh; 1313b-projecting strut; 1314-graft; 1315-radiopaque element; 13151-mounting stud; 13152-radiopaque body; 120-radiopaque ring; A1-first mesh; A2-second mesh; A3-transition mesh; 300-delivery device; 301-sheath; 302-delivery catheter; 500-aspiration device; 501-aspiration tube; 502-coupling base; 503-sealing valve for preventing blood leakage; 504-one-way valve; 10-guide wire; 20-blood vessel; 30-thrombus.

### DETAILED DESCRIPTION

Objects, advantages and features of the present invention will become more apparent upon reading the following more detailed description with reference to the accompanying drawings, which illustrate particular embodiments thereof. Note that the figures are provided in a very simplified form not necessarily drawn to exact scale for the only purpose of helping to explain the disclosed embodiments in a more convenient and clearer way. In addition, the illustrated structures are usually part of their real-world counterparts. In particular, as the figures tend to have distinct emphases, they are sometimes drawn to different scales. Further, it is understood that, as used herein, the terms "first", "second", "third" and the like are only meant to distinguish various components, elements, steps, etc. from each other and are not intended to indicate logical or sequential orderings thereof, unless otherwise indicated or specified.

As used herein, the terms "proximal end" and "distal end" describe relative orientations, positions and directions of components of a piece of medical equipment or actions taken thereon, as viewed by a surgeon operating the medical equipment. Without wishing to be limiting, "distal end" usually refers to an end of the medical equipment that enters the body of a patient first, and "proximal end" to an end closer to the surgeon, during normal operation of the equipment. In the context herein, "distal end" and "proximal end" refer to relative locations, rather than particular ends. For instance, a "distal end" of a capture unit refers to a location near the distal end thereof, rather than exactly the distal end. As used herein, the term "axial direction" usually refers to a direction along a central axis, e.g., of a capture unit or supporting member. "Radial direction" usually refers to a direction perpendicular to an axial direction. "Circumferential direction" refers to a direction about a central axis. "Inner" refers to a location closer to a central axis of a capture unit. "Outer" refers to a location farther away from a central axis of a capture unit. "Leading end" refers to a location closer to a foldable and bendable portion and a leading end of a capture unit is equivalent to "distal end", while "trailing end" refers to a location farther away from the foldable and bendable portion than the leading end. As used herein, a "target lumen" may be a vascular lumen, or a non-vascular lumen such as the lumen of a bile duct or ureter.

It is a principal object of the present invention to provide a medical device and a medical system, which overcome the problems with conventional devices, including low treatment efficiency and low treatment success, when they are used to treat occlusive objects such as thrombi and stones. For example, when treating a thrombus, the present invention provides better penetration and capture of the thrombus and a reduced risk of escape of disrupted thrombus during withdrawal. Moreover, it enables effective removal and collection of an adherent thrombus during transport and easier retraction into a catheter and offers other advantages.

The present invention will be described below with reference to the accompanying drawings. In the following, should there be no conflict, the embodiments described hereunder and features thereof can complement or be combined with each other or one another.

### <Embodiment 1>

Description is set forth below in the context of removing a thrombus from a blood vessel. However, those skilled in the art can adapt the following description to the removal of a stone or other occlusive object through appropriately modifying the details given below.

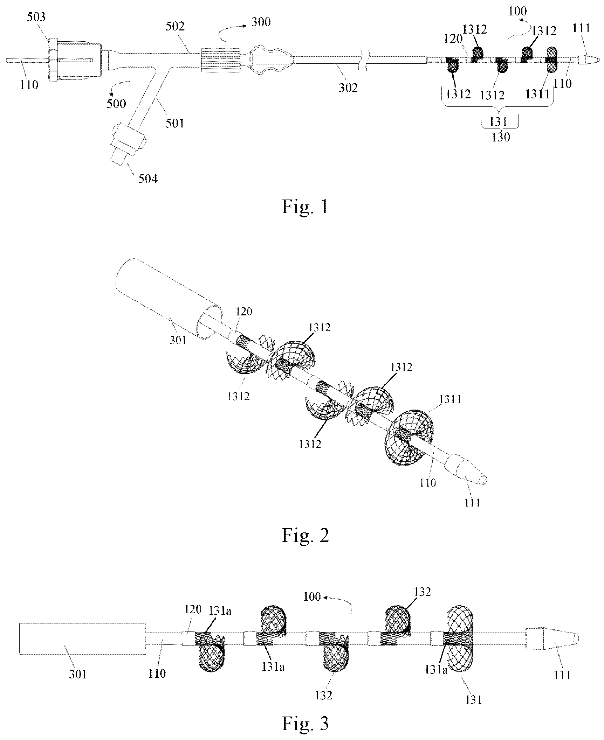

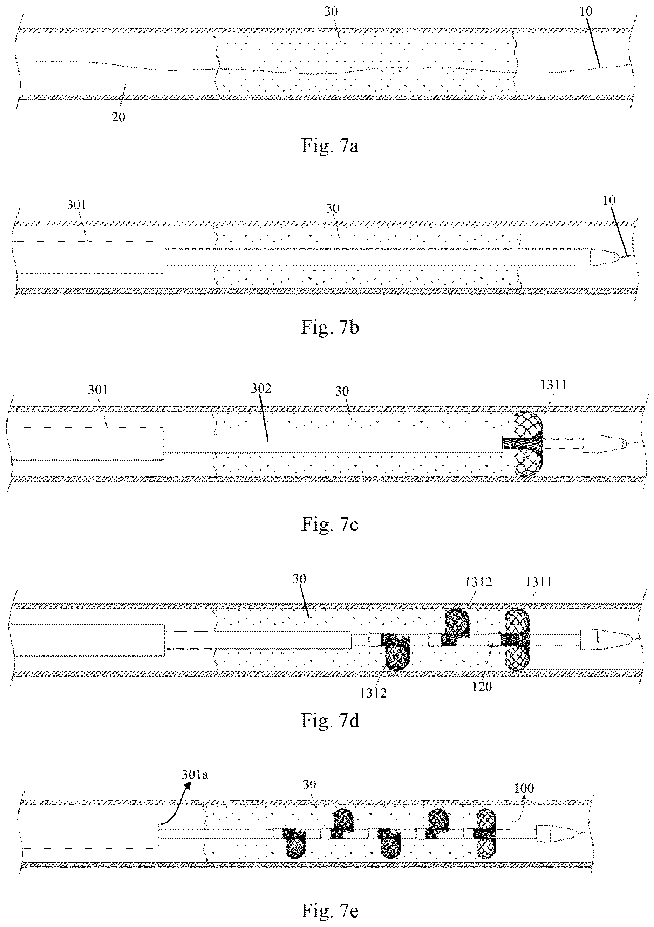

As shown in Figs. 1 to 3, in a first embodiment of the present invention, there is provided a medical system including a medical device 100, a delivery device 300 and an aspiration device 500. The medical device 100 includes a supporting member 110 and a mesh capture device 130. The mesh capture device 130 is mounted over an outer circumference of the supporting member 110 at a distal end thereof and configured to capture, transport and extract a thrombus. The supporting member 110 is generally an elongate tube, and a guide wire 10 may be passed into the interior thereof (see Figs. 7a to 7f). The supporting member 110 is mainly used to support and mount the mesh capture device 130. The supporting member 110 can also be manipulated to release and withdraw the mesh capture device 130.

The mesh capture device 130 includes a plurality of capture units 131 spaced apart along an axis of the supporting member 110 at equal or unequal intervals. Here, those skilled in the art may determine the distances between the capture units 131, in particular according to properties of a target lumen and an occlusive object. Generally, larger distances between adjacent capture units 131 lead to higher compliance of the medical device 100.

In the illustrated implementation, the plurality of capture units 131 are discrete elements, which are all separately attached to the supporting member 110 without being integrally connected to form a single structure, and the formed mesh capture device 130 is accordingly of a split type. In this case, each of the capture units 131 may be formed of a separate single mesh tube and then separately mounted to the supporting member 110. According to the present invention, such mesh tubes may be cut or braided ones, with cut mesh tubes being more preferred because of higher radial strength of the resulting capture units.

In another implementation, the capture units 131 are integrally connected to from a single structure, and the mesh capture device 130 is accordingly of a one-piece or integral type. In this case, all the capture units 131 may be made of a single mesh tube.

In both cases, there are no fewer than three capture units 131, including a first capture unit 1311 and multiple second capture units 1312. The first capture unit 1311 is located farther away from a proximal end of the supporting member 110 than all the second capture units 1312. Generally, there is only one first capture unit 1311 configured for disruption, capture, trapping and transport of a thrombus at the distal end. There may be two or more (i.e., at least two) second capture units 1312 configured for disruption, capture, trapping and transport of a thrombus at the proximal end.

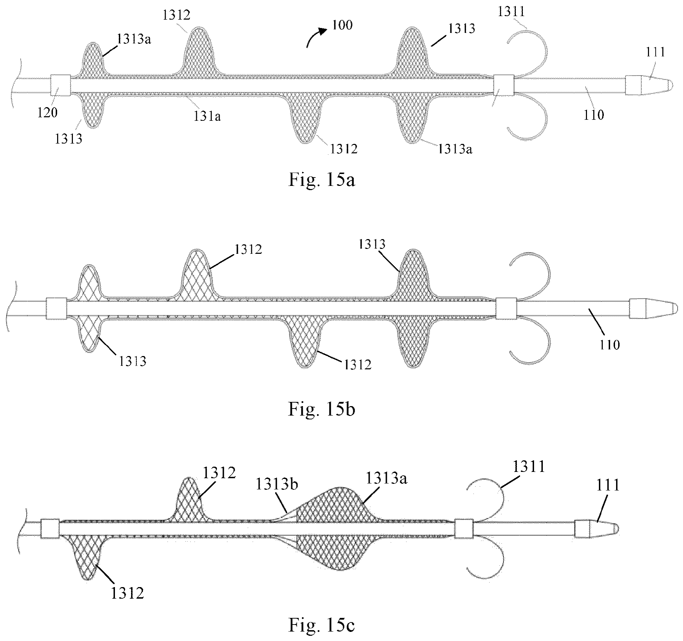

The plurality of capture units 131 may also include a third capture unit 1313 (see Figs. 15a to 15c). In this case, the first capture unit 1311 is farther away from the proximal end of the supporting member 110 than all the third capture unit 1313, and the relative position between the third capture unit 1313 and the second capture units 1312 is not specifically constrained. There may be one or more third capture units 1313. The first capture unit 1311 is structurally different from the second capture units 1312 and from the third capture unit(s) 1313. The third capture unit(s) 1313 is/are structurally different from the second capture units 1312. All the second capture units 1312 may be of the same structure, or not. In the case of plurality of third capture units 1313, the plurality of third capture units 1313 may be of the same structure, or not. As used herein, the term "structure" comprises shape and size, e.g., different in shape or size, or different in both shape and size.

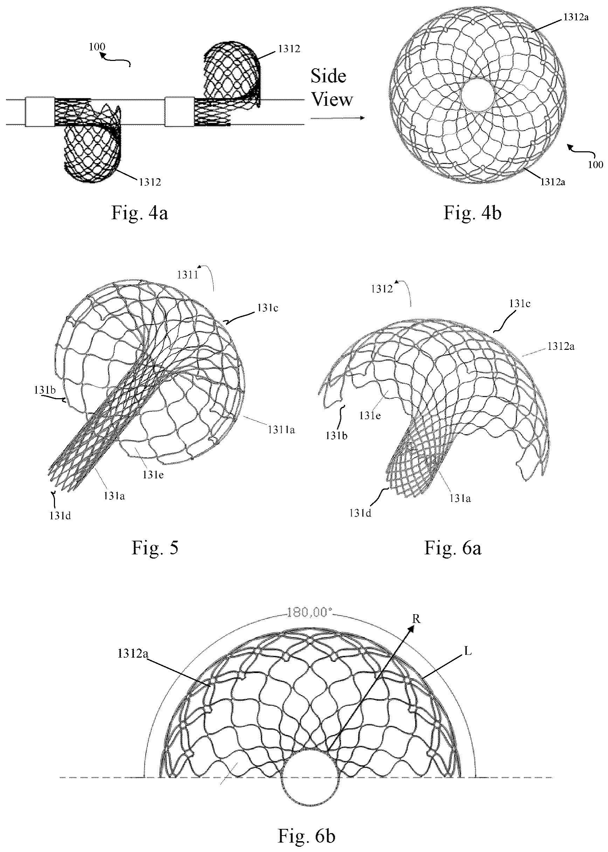

Referring to Figs. 5 and 6a to 6b, each of the first capture unit 1311 and the second capture units 1312 has, when fully expanded, a straight loading section 131a and a capture mesh (i.e., a first capture mesh 1311a or a second capture mesh 1312a) integrally joined along the aforementioned axis. The straight loading section 131a can be sleeved over the outer circumference of the supporting member 110, and the capture mesh is arranged to outwardly folded around the straight loading section 131a and is curved toward the proximal end of the capture unit 131. As shown in Fig. 5, when fully expanded, the first capture unit 1311 has first capture mesh 1311a which is outwardly folded along its entire circumference and curved toward a proximal end of the first capture mesh 1311a, the first capture mesh 1311a is a complete umbrella-shaped surface. As shown in Figs. 6a and 6b, when fully expanded, each second capture unit 1312 has a second capture mesh 1312a which is outwardly folded along part of its circumference and curved toward a proximal end of the second capture units 1312, the second capture mesh 1312a is a partial umbrella-shaped surface.

Each of the first capture unit 1311 and the second capture units 1312 may be made of a separate mesh tube. In order to fabricate the first capture unit 1311, a portion of a mesh tube may be overall folded outwardly to form the first capture mesh 1311a, with the remaining unfolded portion serving as the straight loading section 131a. In order to fabricate the second capture unit 1312, a portion of another mesh tube may be partially folded outwardly to form the second capture mesh 1312a, with the remaining unfolded portion serving as the straight loading section 131a. Desirably, a transition region between the straight loading section 131a and capture mesh of each capture unit is as smooth as possible to prevent stress concentration. For example, the first capture mesh 1311a and the straight loading section 131a of the first capture unit 1311 may have a curved or linear transition. Similarly, the second capture mesh 1312a and the straight loading section 131a of the second capture unit 1312 may also have a curved or linear transition.

According to the present invention, the design combining the first capture unit 1311 with the plurality of second capture units 1312 can not only improve therapeutic efficiency and therapeutic outcomes, but can also increase the compliance and adaptation of the resulting medical device 100 in tortuous blood vessels and blood vessels with a varying diameter. In particular, the partially curved design of the second capture units 1312 can even increase the compliance and adaptation of the device, reducing the likelihood of the device experiencing excessive deformation or inward collapse inside a tortuous blood vessel or a blood vessel with a varying diameter. Moreover, it can better guide the capture units 131 into the delivery device 300, reducing resistance encountered during retraction into a catheter and facilitating delivery and withdrawal. It will be understood that the outwardly folded and curved structural design enables a smaller contact area of the capture units 131 with a wall of a blood vessel, reducing the probability of damage caused to the blood vessel. Further, it enables reduced damage to a blood vessel wall during removal of a thrombus, causing less injury to a patient who receives such treatment.

More specifically, when the capture unit 131 self-expands, a thrombus can be collected within the curved capture mesh, which may be then sucked away by the aspiration device 500. In particular, when the capture meshes are in close contact with an inner wall of a blood vessel, the capture meshes of the capture units 131, when retracted, can effectively "scrape" off a thrombus that adheres to the blood vessel. That is, the device has improved thrombus removal performance because when retracted in a blood vessel, it can easily detach a thrombus firmly adhering to the blood vessel and then capture and transport it away. Apart from these, during the self-expansion of the capture units 131, the capture meshes can cut and disrupt large thrombus during the outwardly folding and curving process, enabling the aspiration device 500 to suck away the fragmented thrombus, thereby improving thrombus removal efficiency.

It will also be recognized that the capture unit 131, when fully expanded, is in the form of a three-dimensional mesh, which may have dense mesh openings and hence good radial strength, which makes the capture unit more resistant to deformation caused by compression by a thrombus during thrombectomy and enables it to effectively cut into, disrupt and capture a thrombus. Moreover, during withdrawal, the captured thrombus is less likely to fall off a surface of the capture unit 131 or escape though the mesh openings thereof. Thus, not only more efficient thrombus capture can be achieved, but the risk of distal thrombotic vascular occlusion can be lowered. In particular, the increased resistance to deformation allows the capture units 131 to more easily extract a harder thrombus from the body, thereby resulting in increased thrombus capture success.

Each of the first capture unit 1311 and the second capture units 1312 is not closed at its trailing end 131b. This enables good compliance and higher resistance to collapse within a tortuous blood vessel or a blood vessel with a varying diameter, reducing the risk of escape or rupture of a captured thrombus. Additionally, the sequentially arrangement of the capture units 131 allows a thrombus to be accommodated between the capture meshes of adjacent capture units 131 making it less likely to escape distally. Further, this enables a thrombus to be disrupted multiple times, providing better thrombus disruption.

Referring again to Figs. 6a and 6b, in conjunction with Fig. 3, outwardly folding and curving second capture unit 1312 along part of its circumference can additionally increase overall compliance of the device, enabling it to behave better in a tortuous blood vessel or a blood vessel with a varying diameter. The present invention is not limited to any particular number of second capture units 1312. Although four second capture units 1312 have been shown in the figures, fewer or more second capture units 1312 are possible in other implementations (e.g., two, three, five, or six second capture units 1312). Preferably, the second capture units 1312 are paired so that the second capture meshes 1312a of adjacent second capture units 1312 span the entire circumference of the supporting member 110, i.e., they form a complete umbrella-shaped surface along the entire circumference. With this arrangement, even better thrombus trapping and disruption and improved thrombus removal performance can be achieved, as can be particularly seen from Figs. 4a and 4b.

As shown in Figs. 4a and 4b, in this embodiment, there are two adjacent second capture units 1312, the second capture meshes 1312a of which are staggered around the circumference of the supporting member 110, enabling the combination of the second capture meshes 1312a of adjacent second capture units 1312 form a complete umbrella-shaped surface around the circumference of the supporting member 110. This not only allows for effective thrombus trapping and capture, but can also increase the compliance and adaptation of the device in tortuous blood vessels and blood vessels with a varying diameter, making the mesh capture units 131 less likely to experience excessive deformation or inward collapse inside a blood vessel. Further, resistance to retraction into a catheter can be reduced, facilitating delivery and withdrawal.

Next, referring to Fig. 6b, in one implementation, when the second capture units 1312 are orthogonally projected in the distal-to-proximal direction, the outermost projection line L of the second capture meshes 1312a of the second capture unit 1312 forms a 180° arc angle relative to the center of the second capture unit 1312. However, the present invention is not so limited, in other implementations, the arc angle of the outermost projection line L of the second capture meshes 1312a may also be greater than or less than 180°, as long as the second capture meshes 1312a of the two second capture units 1312form a complete umbrella-shaped surface along the entire circumference of the supporting member 110. The second capture mesh 1312a of each second capture unit 1312 may be curved at any appropriate angle, but the arc angle of the outermost projection line L of the second capture meshes 1312a of the second capture unit 1312 is always less than 360°.

In a surgical procedure, the medical device 100 is delivered into the body of a patient with the aid of the delivery device 300. The delivery device 300 loads the medical device 100. Specifically, the medical device 100 may be crimped into the delivery device 300 and carried thereby to a target lesion site. Of course, apart from delivering the medical device 100, the delivery device 300 may provide other functions, such as controlling release and withdrawal of the medical device 100. For more efficient and better thrombectomy, assistance from the aspiration device 500 is often necessary, which is provided by sucking a thrombus into the aspiration device 500 and extracting it out of the body. That is, the aspiration device 500 is used to aspirate away a thrombus captured by the medical device 100. The aspiration device 500 may be coupled to the delivery device 300 either detachably or non-detachably. The aspiration device 500 is configured to apply a suction force through the delivery device 100 to suck a thrombus away from the target lesion site. Those skilled in the art can understand the structures and functions of the delivery device 300 and the aspiration device 500 based on the knowledge in the art, and more detailed description thereof is omitted herein.

In the illustrated implementation, the mesh capture device 130 is a split configuration, the straight loading sections 131a of the capture units 131 are substantially cylindrical tubes, all of which can be sleeved over the outer circumference of the supporting member 110 at the distal end thereof and then fixedly attached to the supporting member 110. As exemplified below, the attachment of the straight loading sections 131a to the supporting member 110 may be accomplished using at least one of various approaches.

The straight loading sections 131a of the capture units 131 may be thermally fused, glued or mechanically attached to the supporting member 110. Here, the mechanical attachment may be accomplished by any method known to those skilled in the art, including, but not limited to, welding. In this embodiment, the straight loading sections 131a of the capture units 131 are fixedly attached to the supporting member 110 through the radiopaque rings 120. The radiopaque rings 120 may be sleeved over the exterior of the straight loading sections 131a so that the proximal ends of the straight loading sections 131a cover and is fixed to the outer circumference of the supporting member 110. The radiopaque rings 120 are made of a radiopaque material, and the present invention is not limited to any particular type of material. The radiopaque rings 120 are used to mark the positions of proximal ends 131d of the capture units 131.

The capture unit 131 is a self-expanding mesh structure, which can be stressed into a crimped configuration and will transition from the crimped configuration into an expanded configuration by itself when not stressed anymore. The capture unit 131 is in its natural configuration when not under any external force (the natural configuration of the capture unit 131 is an expanded configuration). When compressed and loaded within delivery device 300 for delivery, the capture unit 131 comprises a crimped configuration. Generally, when in the crimped configuration, the capture unit 131 comprises a substantially linear shape extending substantially parallel to the supporting member 110. In other words, when in the crimped configuration, the umbrella-shaped surface of the capture unit 131 is inverted so as to be almost collinear with the straight loading section 131a. This enables a reduced size during delivery. After released from the delivery device 300, the capture unit 131 can expand to comprise the expanded configuration. Each capture unit 131 is switchable between the crimped and expanded configurations.

The capture units 131 are typically made of a metallic material, such as a metal or alloy. A metallic material with shape memory properties is particularly preferred, examples of which may include, but are not limited to, nickel-titanium alloys. Metal mesh tubes may be prepared using various methods known in the art and then subjected to a shape setting process, obtaining the capture units 131 each having a straight loading section 131a and a capture mesh integral therewith. As well known in the field of heat setting, molds can be used to maintain the metal mesh tubes in desired final shapes, and appropriate heat treatment may be then applied to fix the desired shapes for the capture units 131.

In some implementations, the capture unit 131 is integrally formed by cutting a metal tube. Suitable cutting methods may include, but are not limited to, laser cutting. In other implementations, the capture unit 131 is integrally braided with multiple metal wires. The present invention is not limited to any particular braiding method. In this embodiment, each of the first capture unit 1311 and second capture units 1312 is fabricated by cutting a metal tube with shape memory properties and then subjecting it to heat treatment for shape-setting. In this way, the resulting capture units 131 exhibit good radial strength and the outwardly folded and curved capture meshes ensure desirable compliance and adaptation of the capture units 131, which enable reduced damage to blood vessel walls, good thrombus detachment, capture and transport performance and reduced resistance to retraction into the delivery device.

The number of capture units 131 may depend on, amongst others, the length of a lesion (e.g., a thrombus) and a desired thrombectomy length for the capture units 131. For example, in the case of thrombus removal, in order to quickly remove a large amount of thrombus, one first capture unit 1311 and more than two second capture units 1312 may be usually provided to remove the thrombus from the body in a segment way. When there are only a small number of thrombi to be removed, one first capture unit 1311 and two second capture units 1312 may be provided. The capture units 131 may have equal or different lengths and radial dimensions.

The present invention is not limited to any particular shape of mesh openings 131e in the capture units 131, and the mesh openings 131e may have any appropriate shape, such as rhombic, diamond-like, rectangular, circular or elliptical shape. Other shapes are also possible. In some implementations, the straight loading sections 131a have substantially rhombic or diamond-like mesh openings 131e, while the capture meshes may have mesh openings 131e of any suitable shape. Possible examples of the shape of the mesh openings 131e in the capture meshes may include rectangular, circular, rhombic, elliptical shape, or a combination thereof.

Each straight loading section 131a may have a substantially constant density of mesh openings, while each capture mesh may have a density of mesh openings which varies as the capture mesh is outwardly folded and curved as to gradually decrease from the center to the periphery of the capture mesh, thereby forming a mesh opening-dense region close to the center and a mesh opening-sparse region away from the center. This can be seen from Fig. 6b, a schematic illustration of the second capture unit 1312. As shown, the mesh opening density of the second capture mesh 1312a of the second capture unit 1312 gradually decreases along the direction R from the center to the periphery. The number of the mesh openings in mesh opening-dense region is greater than the number of the mesh openings in the mesh opening-sparse region, but the size (diameter) of the mesh opening in the mesh opening-dense region are smaller (in diameter) than the size of the mesh opening in the opening-sparse region. The mesh opening-dense region has better thrombus collecting performance and is associated with a reduced probability of distal thrombus escape. In contrast, the mesh opening-sparse region facilitates disrupting the large thrombus during outward folding, expansion and curving of the capture unit 131, or during retraction thereof. This configuration of the mesh openings with denser mesh openings around the center and sparser mesh openings along the periphery enables both good thrombus retention and reduced resistance to sheathing.

In practical use, at least one of the capture units 131, when fully expanded, has a radial dimension greater than or equal to a diameter of the diseased blood vessel. This enables the capture mesh of the capture unit 131 to effectively scrape off a thrombus or thrombi adhering to an inner wall of the blood vessel while being movable along the inner wall of the blood vessel to collect disrupted thrombus in the umbrella-like capture mesh and allowing blood to flow therethrough during withdrawal. Ensuring smooth blood flow is meaningful because the patient's life may be otherwise threatened. In addition, when in the fully expanded configuration, a maximum diameter of the capture mesh in the capture unit 131 or a diameter of another portion thereof may be greater than or equal to the diameter of the diseased blood vessel.

Referring to Figs. 1 to 3, a distal guide head 111 is provided at a distal end of the supporting member 110. A distal portion of the distal guide head 111 has a smooth surface, which causes almost no damage to surrounding tissue. The distal guide head 111 is provided to facilitate advancement of the medical device 100 within a lumen. The distal guide head 111 may be a hollow cone with a spherical distal end, and there is a step at the junction of the cone and the supporting member 110. The step may come into abutment with a distal end face of a delivery catheter 302 of the delivery device 300 and be thereby blocked from entering the interior of the delivery catheter 302. In practice, the supporting member 110 may be manipulated to release the medical device 100 by pushing it out of the delivery device 300. If the medical device 100 is released at a wrong location, it may be retracted back into the delivery device 300 and then again released after location adjustment. After thrombectomy, the supporting member 110 may be again manipulated to retract the medical device 100 back into the delivery device 300 and then withdraw the thrombus out of the body.

In one implementation, the supporting member 110 further includes a hollow core preferably having an elastic section and a stiff section arranged along the axial direction from the distal end to the proximal end. The elastic section is softer than the stiff section. The elastic section is configured for mounting the mesh capture device 100. The elastic and stiff sections may be integrally formed with each other, or separately formed and then assembled together, for example by coupling them to each other. A distal end of the stiff section may be coupled to a proximal end of the elastic section by heat welding, snap engagement or another known technique, and the present invention is not limited to any particular method for coupling. The stiff section is more capable of force transfer, and the elastic section is freely stretchable, retractable and bendable in response to bending of the capture units 131, increasing the compliance of the medical device 100 and making it suitable for use in tortuous blood vessels and blood vessels with a varying diameter. In other implementations, the entire hollow core may be stiff.

As shown in Figs. 1 to 3, the delivery device 300 typically includes an outer sheath 301 and an inner delivery catheter 302 arranged coaxially with the outer sheath 301. The supporting member 110 is inserted through the delivery catheter 302. The delivery catheter 302 is able to deliver the capture units 131 all in the crimped configuration to a diseased site. The delivery catheter 302 is inserted through the sheath 301 and can be advanced therein. After thrombectomy, the medical device 100 can be retracted back into the sheath 301. The proximal end of the sheath 301 is sealed with a sealing valve to prevent leakage of blood from the proximal end of the sheath 301.

As shown in Fig. 1, the aspiration device 500 includes an aspiration tube 501 and a coupling base 502. The aspiration device 500 may also include an aspiration device (not shown). The coupling base 502 is detachably disposed at a proximal end of the delivery catheter 302, and a sealing valve 503 for preventing blood leakage is often arranged at an open proximal end of the coupling base 502 to sealingly close the proximal end of the coupling base 502. The proximal end of the delivery catheter 302 is coupled to the coupling base 502 to form a main passage, through which the medical device 100 can be moved into and out of the delivery catheter 302. The aspiration tube 501 is disposed on one side of the coupling base 502 and distal end of the aspiration tube 501 is connected to a lateral hole in the coupling base 502. The proximal end of the aspiration tube 501 is often provided with a one-way valve 504 configured for connection with the aspiration device. The aspiration device is able to apply a suction force to the delivery catheter 302 through the aspiration tube 501. The aspiration device may be selected from any possible fluid delivery device, such as a vacuum pump or the like.

Given that some old thrombi exhibit tight adhesion to the vascular wall, and considerable effort may be required for the capture units 131 to scrape off or penetrate such thrombi. In order to overcome this problem, in one implementation, the delivery catheter 302 is provided with a plurality of micropores, through which a thrombolytic fluid can be sprayed and released to dissolve the thrombi. The micropores may be evenly or unevenly arranged along an axis of the delivery catheter 302 or around a circumference thereof. Alternatively, the micropores may be arranged both along the axis of the delivery catheter 302 and around its circumference. The thrombolytic fluid will lyse or soften thrombus portions that it contacts, enabling a capture unit 131 to more efficient scrape off or penetrate the thrombus, resulting in higher old thrombus removal success. In other implementations, some thrombolytic agent may be sprayed on the capture meshes of the capture units 131 in the form of crystals covered on the capture meshes, which allow for rapid release of the agent. As the capture units 131 expand, the capture meshes will be outwardly folded and curved to surround a thrombus or thrombi and come into contact with a blood vessel wall. At a later time, the thrombolytic agent will lyse or soften thrombus portions that it contacts, enabling the capture meshes to more efficiently scrape off or cut into the thrombus or thrombi. At this point, the medical device may be withdrawn. In this way, higher old thrombus removal success can also be obtained. These approaches, i.e., releasing a thrombolytic fluid through the micropores and spaying a thrombolytic agent on the capture units 131, may be used separately or in combination.

Next, a process to use the medical system 100 is further described with reference to Figs. 7a to 7f in the context of removing a thrombus from a blood vessel as an example.

As shown in Fig. 7a, in order to remove the thrombus from a diseased site inside the blood vessel, a guide wire 10 is first advanced through the blood vessel 20 to the thrombus 30 and then pushed through the entire thrombus 30.

As shown in Fig. 7b, the sheath 301 is advanced over the guide wire 10 to a location near the thrombus 30 and then be held still there, and the delivery catheter 302 with the medical device 100 being loaded therein is then advanced also over the guide wire 10 until the distal guide head 111 at the distal end of the supporting member 110 completely passes through the thrombus 30.

Subsequently, as shown in Figs. 7c to 7e, with the supporting member 110 being held stationary, the delivery catheter 302 is gradually retracted until the plurality of capture units 131 successively fully expand, the capture meshes of the capture units 131 adequately penetrate into and disrupt the thrombus 30. The aspiration device is then activated to suck the thrombus at a distal opening 301a of the sheath 301 into lumen of the delivery catheter 302 and into the aspiration tube 501.

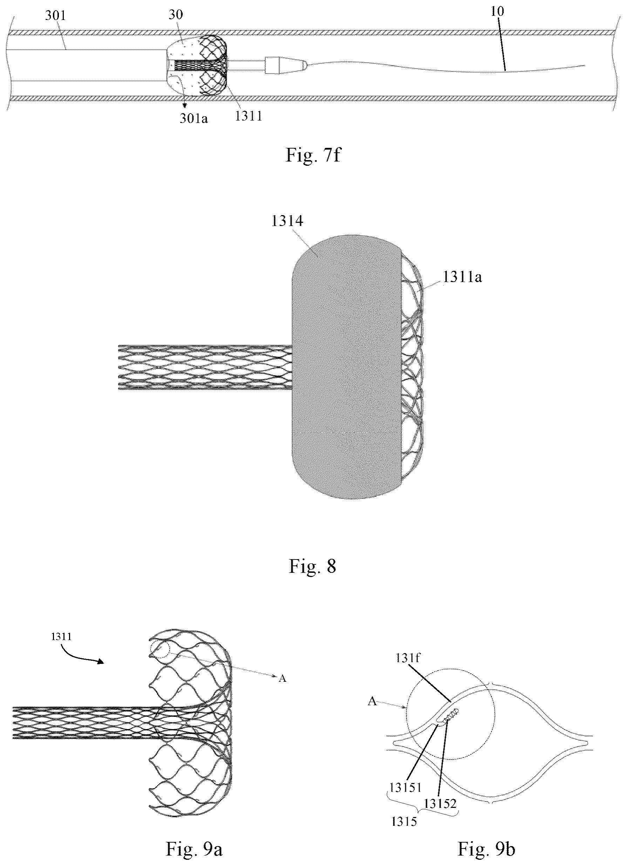

As shown in Fig. 7f, after all the capture units 131 expand, the supporting member 110 is retracted to the proximal end, and hence all the capture units 131 is slowly retracted to the proximal end, the thrombus 30 is gradually peel off, squeezed, cut and disrupted and retained between the capture mesh of capture unit 131 and adjacent capture unit 131, and the capture units 131 and the supporting member 110 are gradually displaced toward the distal opening 301a of the sheath 301. In this process, the aspiration device continually aspirates the thrombus 30 until the last (most distal) capture unit 131 is retracted into the sheath 301. At this point, the thrombus will be partially collected in the sheath 301. In this way, the whole thrombus will be eventually extracted out of the body together with the medical device 100.

Preferably, a mesh opening density of the plurality of capture units 131 gradually decreases along the distal-to-proximal direction. Thus, a more proximal capture unit 131 has sparse mesh openings 131e, which facilitate disruption of large thrombus fragments. Additionally, a more distal capture unit 131 has denser mesh openings 131e, which help trap finer thrombus and prevent their distal escape. However, in other implementations, the plurality of capture units 131 may be consistent in mesh opening density.

### <Embodiment 2>

Referring to Fig. 8, capture units 131 according to a second embodiment of the present invention are substantially similar to those of the first embodiment in terms of structure, except for some differences. In the following, only these differences will be described, and the similarities between the two embodiments are not repeated.

As shown in Fig. 8, differing from the first embodiment, the capture units of the second embodiment include a first capture unit 1311 having a first capture mesh 1311a provided with a graft 1314, which covers some mesh openings 131e in the first capture mesh 1311a. Preferably, the graft 1314 covers only an opening-sparse region of the first capture mesh 1311a, but not an opening-dense region thereof. With the graft 1314, the first capture unit 1311 is able to better collect and block disrupted thrombus from escape and thereby provide improved thrombectomy performance, while allowing smooth blood flow in the lumen. The graft 1314 may be disposed either on an inner surface or on an outer surface of the first capture mesh 1311a. The graft 1314 may be sewn, or otherwise fixedly attached to, the first capture mesh 1311a.

### <Embodiment 3>

Referring to Figs. 9a and 9b, capture units 131 according to a third embodiment of the present invention are substantially similar to those of the first embodiment in terms of structure, except for some differences. In the following, only these differences will be described, and the similarities between the two embodiments are not repeated.

As shown in Figs. 9a and 9b, differing from the first embodiment, the capture units 131 of the third embodiment includes a capture mesh provided with a radiopaque element 1315 disposed on or near a mesh strut 131f. The radiopaque element 1315 is visible on X-ray images, thereby providing an operator with a basis for observing the location of the capture unit 131 within a lumen and its extent of expansion in real time during a surgical procedure. Although the radiopaque element 1315 has been illustrated as being provided on a first capture unit 1311, in fact, a second capture unit 1312 may also be provided with such a radiopaque element 1315.

Preferably, the radiopaque element 1315 is disposed at a location comprising a maximum radial dimension of the capture mesh in an expanded configuration. This allows better observation of the contact between the capture mesh and a wall. Preferably, a plurality of radiopaque elements 1315 are provided on a single circumference. However, those skilled in the art will recognize that the radiopaque elements 1315 may be indeed arranged at any appropriate locations on the capture mesh in various patterns, without departing from the scope of the present invention. Additionally, the present invention is not limited to any particular approach for arranging the radiopaque elements 1315 on the capture mesh, and those skilled in the art can selected at least one of various possible approaches. Some examples are given below. One such example is set forth below.

In one implementation, as shown in Fig. 9b, each radiopaque element 1315 includes a mounting stud 13151 and a radiopaque body 13152 on the mounting stud 13151. The mounting stud 13151 is located within a mesh opening 131e, with its one end being connected to a mesh strut 131f. The other end of the mounting stud 13151 is free. The radiopaque body 13152 may be implemented as any radiopaque structure, such as a radiopaque wire, a radiopaque ring or the like. In the illustrated example, the radiopaque body 13152 is a radiopaque wire spirally wound on the mounting stud 13151. The mounting stud 13151 may be integrally formed with the mesh tube, for example, by cutting. This allows for easy fabrication. The radiopaque body 13152 may be made of a common radiopaque material, examples of which may include, but are not limited to, a platinum-iridium alloy, tungsten, etc. The radiopaque body 13152 may be fixedly attached to the mounting stud 13151 in various manners, such as welding, gluing or snap engagement. In other implementations, the radiopaque elements 1315 may be replaced with radiopaque structures made of composite wires (e.g., DFT^{®}), the composite wire includes a nickel-titanium sleeve and a radiopaque core wire surrounded by the nickel-titanium sleeve.

It should be noted that although this embodiment has been described in the context of a mounting stud 13151 and a radiopaque body 13152, as will be appreciated by those skilled in the art, the present invention is not so limited. In some alternative embodiments, the radiopaque elements 1315 may be directly implemented as radiopaque sleeves disposed over mesh struts 131f, or as radiopaque wires wound on mesh struts 131f.

### <Embodiment 4>

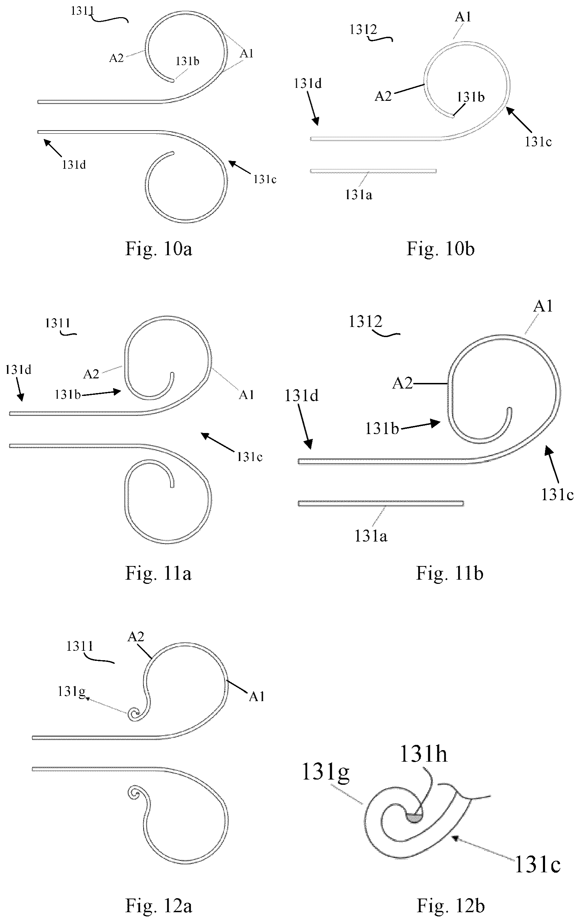

Referring to Figs. 10a to 10b, 11a to 11b, 12a to 12c and 13a to 13b, capture units 131 according to a fourth embodiment of the present invention are substantially similar to those of the first embodiment in terms of structure, except for some differences. In the following, only these differences will be described, and the similarities between the two embodiments are not repeated. It should be noted that shown in the figures are simplified schematics of the capture units 131, which show only contours of them, but not details of their mesh structures.

Differing from the first embodiment, capture meshes of the capture units 131 of the fourth embodiment can have different degrees of curvatures and different curved shapes.

In this embodiment, each of the first capture mesh 1311a and the second capture meshes 1312a has a leading end 131c and a trailing end 131b, which oppose each other along an axis thereof. The leading end 131c is curved toward the proximal end, and the trailing end 131b is curved toward the leading end 131c.

As shown in Fig. 10a, in an exemplary implementation, the first capture mesh 1311a of the first capture unit 1311 has an inner first mesh A1 and an outer second mesh A2 directly joined to the first mesh A1. The first mesh A1 forms the leading end 131c of the first capture mesh 1311a, and the second mesh A2 forms the trailing end 131b of the first capture mesh 1311a. In a fully expanded configuration of the first capture unit 1311, the first mesh A1 is curved toward a proximal end 131d of the first capture unit 1311, and the second mesh A2 is curved toward the first mesh A1, so that the trailing end 131b of the first capture unit 1311 is located within the umbrella -like first capture mesh 1311a and is oriented toward the first mesh A1. With this arrangement, during retraction, a considerable proportion of resistance to the retraction will be shared by the second mesh A2, lowering the likelihood of the curved meshes being undesirably distally flipped in the event of a large amount of thrombus and making the first capture mesh 1311a of the first capture unit 1311 more resistant to deformation. In the illustrated example, both the first mesh A1 and the second mesh A2 of the first capture unit 1311 are arcuate, and they oppose each other along a direction of the thrombectomy, and a direction of the thrombectomy corresponds to an axis of the first capture unit 1311.

As shown in Fig. 10b, similar to the first capture unit 1311, in the second capture unit 1312, the second capture mesh 1312a may have an inner first mesh A1 and an outer second mesh A2 directly joined to the first mesh A1. The first mesh A1 forms the leading end 131c of the second capture units 1312, and the second mesh A2 forms the trailing end 131b of the second capture unit 1312. In a fully expanded configuration of the second capture unit 1312, the first mesh A1 is curved toward a proximal end 131d of the second capture unit 1312, and the second mesh A2 is curved toward the first mesh A1, so that the trailing end 131b of the second capture unit 1312 is located within the umbrella-like second capture mesh 1312a and is oriented toward the first mesh A1. With this arrangement, during retraction, a considerable proportion of resistance to the retraction will be shared by the second mesh A2 of the second capture unit 1312, lowering the likelihood of the curved meshes being undesirably distally flipped in the event of a large amount of thrombus material and making the second capture mesh 1312a of the second capture unit 1312 more resistant to deformation. In the illustrated example, both the first mesh A1 and the second mesh A2 of the second capture unit 1312 are arcuate, and they oppose each other along a direction of the thrombectomy.

As shown in Fig. 11a, in another exemplary implementation, the first capture mesh 1311a of the first capture unit 1311 is a combination of an arcuate mesh and a polygonal mesh. Specifically, in the first capture unit 1311, the arcuate mesh is a first mesh A1, and the polygonal mesh is a second mesh A2. There is a smooth transition between the first mesh A1 and the second mesh A2. As shown in Fig. 11b, similar to the first capture unit 1311, in this exemplary implementation, the second capture mesh 1312a of each second capture unit 1312 may be a combination of an arcuate mesh and a polygonal mesh. Specifically, in each second capture unit 1312, the arcuate mesh is a first mesh A1, and the polygonal mesh is a second mesh A2. There is a smooth transition between the first mesh A1 and the second mesh A2.

As would be appreciated, the second mesh A2 functions primarily to enhance supporting and trapping capabilities of the capture mesh, further the capture unit has better supporting and trapping capabilities, resulting in improved removal performance. The mesh opening densities at the trailing end 131b and the leading end 131c may be same or different. Preferably, the mesh opening density at the trailing end 131b is lower than that at the leading end 131c. With this arrangement, enabling the trailing end 131b to cut, disrupt, and trap thrombus around the near side of the capture mesh and enabling the leading end 131c to further trap thrombus around the far side of the capture mesh. Accordingly, during retraction, larger mesh openings 131e at the trailing end 131b can better cut and disrupt large thrombus, and denser mesh openings 131e at the leading end 131c can trap finer thrombus particles and block their escape.

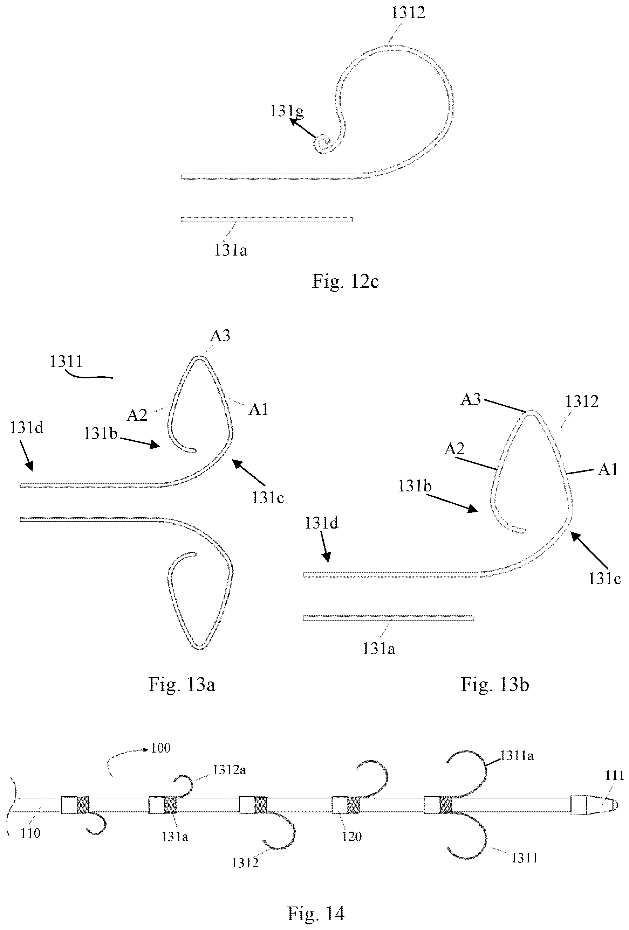

As shown in Figs. 12a and 12b, after fully expanded, the first capture unit 1311 is preferably curved outwards at the trailing end 131b to form a non-invasive end 131g (as a non-limiting example, in the form of a circular ring). The curvature of the non-invasive end 131g may be configured as desired, for example, a single loop, multiple loops, or less than a loop. With the configuration of the non-invasive end 131g, the damage caused by the trailing end 131b to a blood vessel during the self-expansion of the first capture unit 1311, or during its retraction into the sheath 301 can be reduced. Preferably, a soft material 131h is applied to a surface of the non-invasive end 131g. The soft material 131h may be a soft polymer material, which can additionally reduce the risk of damage caused to a blood vessel by the trailing end 131b. As shown in Fig. 12c, the second capture unit 1312 may be similarly curved outwards at the trailing end 131b to form a non-invasive end 131g, and a soft material 131h may also be applied to the non-invasive end 131g.

As shown in Fig. 13a, in yet another exemplary implementation, in the first capture unit 1311, a first mesh A1 and a second mesh A2 are both polygonal mesh and are connected by a transition mesh A3 between them. The transition mesh A3 is an arcuate mesh and provides a smooth transition between the two polygonal meshes. The polygonal first mesh A1 is used as the leading end 131c, and the polygonal second mesh A2 forms the trailing end 131b. The arcuate transition mesh A3 is configured to come into contact with an inner wall of a blood vessel and provide support. This enables a capture mesh with good support, and as the transition mesh A3 has a small contact area with the blood vessel, the damage caused to the blood vessel during thrombus removal can be further reduced. As shown in Fig. 13b, the second capture mesh 1312a of the second capture unit 1312 may be structured in a similar way and, therefore, need not be described in further detail herein.

Therefore, each of the first capture unit 1311 and the second capture units 1312 may have an arcuate or polygonal mesh at the leading end 131c and may also have an arcuate or polygonal mesh at the trailing end 131b.

However, the present invention is not limited to the degrees of curvature or curved shapes of the capture units 131 in the above implementations, and those skilled in the art can appropriately design the degrees of curvature and curved shapes of the capture units 131 as desired in practical applications. These degrees of curvature and curved shapes of the capture units 131 can be obtained by heat setting processes using different molds. A mesh capture device 130 according to this embodiment may include capture units 131 of one or more of the types described above.

### <Embodiment 5>

Referring to Fig. 14, a medical device 100 according to a fifth embodiment of the present invention is substantially similar to that of the first embodiment in terms of structure, except for some differences. In the following, only these differences will be described, and the similarities between the two embodiments are not repeated. It should be noted that Fig. 14 is a simplified schematic, which show only contours of capture units 131, but not details of their mesh structures.

As shown in Fig. 14, in the medical device 100 of the fifth embodiment, a distal end of a supporting member 110 is provided with a plurality of capture units 131 including one first capture unit 1311 and four second capture units 1312. Each pair of the second capture units 1312 can form a complete umbrella-shaped surface. In addition, a mesh capture device 130 according to this embodiment has a radial dimension (corresponding to a diameter after fully expanded) gradually decreasing from a distal end to a proximal end. The first capture unit 1311, which is the most distal one of the capture units 131, has a radial dimension preferred to be greater than or equal to a diameter of a diseased blood vessel. This enables close contact of the most distal first capture unit 1311 with an inner wall of the blood vessel. It should be understood that the radial dimension of the complete umbrella-shaped surface formed by the second capture meshes 1312a of adjacent second capture units 1312 is considered as whole, the radial dimension defined by adjacent second capture units 1312 varies axially with respect to the supporting member 110.

Such a structural design with a varying radial dimension helps reduce resistance from thrombus to the proximal capture units 131 during retraction, thereby minimizing excessive deformation or collapse of the proximal capture unit 131 caused by excessive thrombus resistance during retraction. Additionally, the proximal capture units 131 can deform inwards when retracted. This can provide a desirable buffer effect, which can reduce possible loss of thrombus during withdrawal. Apart from these, the varying radial dimension enables the mesh capture device 130 to exhibit increased compliance and adaptation in tortuous blood vessels and blood vessels with a varying diameter.

However, in alternative exemplary embodiments, the mesh capture device 130 may have a radial dimension gradually increasing from the distal end to the proximal end. In these cases, the most proximal one of the second capture units 1312 may have a radial dimension preferred to be greater than or equal to a diameter of a diseased blood vessel. This enables close contact of the most proximal second capture unit 1312 with an inner wall of the blood vessel. This arrangement can also increase the compliance and adaptation of the mesh capture device 130 in tortuous blood vessels and blood vessels with a varying diameter. In particular, it allows the distalmost first capture unit 131 to be inserted into a narrow blood vessel (e.g., a branch vessel in the lungs) to remove thrombus or thrombi therein with reduced likeliness of causing damage to the blood vessel.

### <Embodiment 6>

Referring to Figs. 15a to 15c, a medical device 100 according to a sixth embodiment of the present invention is substantially similar to that of the first embodiment in terms of structure, except for some differences. In the following, only these differences will be described, and the similarities between the two embodiments are not repeated.

As shown in Figs. 15a to 15c, in the medical device 100 of the sixth embodiment, the mesh capture device 130 is an integral or one-piece device and a plurality of capture units 131 are mutually connected as a single structure. The mesh capture device 130 is sleeved over a supporting member 110, as a whole. The mesh capture device 130 may be formed from a single mesh tube, such as a braided or cut tube that has undergone a heat setting process.

In this embodiment, adjacent capture units 131 are directly connected by straight loading sections 131a. That is, the straight loading sections 131a are commonly shared by adjacent capture units 131. The second capture mesh 1312a is formed by directly radial raising of the mesh tube along a part of a circumference thereof. Thus, the second capture mesh 1312a resemble mesh hump. Preferably, the second capture meshes 1312a of two adjacent second capture units 1312 are non-curved capture meshes along the entire circumference. The second capture meshes 1312a that are each radially raised along part of the circumference enable the device to have increased compliance and reduce its likelihood of excessive deformation or inward collapse within a curved blood vessel, making it suitable for use in tortuous blood vessels and blood vessels with a varying diameter. This design can also reduce resistance to retraction into a catheter, facilitating delivery and withdrawal.

In this embodiment, the second capture mesh 1312a may have any of various appropriate shapes, such as a shape defined by portion(s) of at least one of a sphere, a disk (or a disk-like shape), a cylinder, a basket and a bag. In the case of the second capture meshes 1312a of two adjacent second capture units 1312 forming a complete circular or approximately circular capture mesh, even better supporting member can be provided, and a thrombus or thrombi inside a blood vessel or adhering to a wall of the blood vessel wall can be effectively removed or scraped off. Moreover, the capture units 131 are less likely to experience excessive deformation or collapse during retraction due to an excessive amount of thrombus. Further, in the case of the second capture meshes 1312a of two adjacent second capture units 1312 forming a complete basket- or bag-like capture meshes, thrombus can be even better collected and prevented from distal escape.

Similar to the first embodiment, the second capture mesh 1312a in this embodiment has an arc angle less than 360°, preferably of 180°.

Referring to Figs. 15a to 15c, the mesh capture device 130 may further include a third capture unit 1313 having a third capture mesh 1313a radially raised along the entire circumference. The third capture mesh 1313a that is radially raised along the entire circumference allows the device to have improved support and trapping capabilities. The third capture mesh 1313a may be formed by a portion of the mesh tube radially raised along the entire circumference thereof. Likewise, the third capture mesh 1313a may also have any of various appropriate shapes, such as a shape defined by at least one of a sphere, a disk, a cylinder, a basket and a bag. The third capture mesh 313a may be deflected proximally or distally, thereby reducing resistance to its retraction into a sheath 302.

Each of the first capture unit 1311 to the third capture unit 1313 has very dense mesh openings 131e. At least one of the capture units 131, after fully expanded, has a radial dimension greater than or equal to a diameter of a diseased blood vessel. This enables good detachment, collection and transport of fine disrupted thrombus. The shapes and numbers of the first, second and third capture units and their diameters in the expanded configuration may be determined depending on the use and conditions in a lumen, and the present invention is not particularly limited in this regard.

As shown in Fig. 15a, in one example, there are two second capture units 1312 and two third capture units 1313. Without limitation, the two second capture units 1312 may be located between the two third capture units 1313. The second capture unit 1312 is defined by a shape of a portion of a disc (having a circular cross-section) and two second capture units 1312 can form a complete disc-shaped capture mesh. The third capture units 1313 are in the shape of discs. The second capture units 1312 and the third capture units 1313 have the same mesh opening density. When expanded, the most proximal one of the third capture units 1313 has a radial dimension less than a radial dimension of any of the remaining capture units 131. Close contact can be attained between the first capture unit 1311, which is the most distal one of the capture units 131, and a wall of a blood vessel.

Differing from the example of Fig. 15a, as shown in Fig. 15b, the second capture units 1312 and the third capture units 1313 have different mesh opening densities, and the capture units 131 exhibit a mesh opening density gradually decreasing from distal to proximal direction. The proximal sparser mesh openings enable better disruption of large thrombus, and the distal denser mesh openings can reduce the likelihood of distal escape of thrombus particles.

As shown in Fig. 15c, in another example, there is a third capture unit 1313 in the shape of a basket. The basket is not limited to being located as shown. The basket-shaped third capture unit 1313 includes projecting struts 1313b at a proximal end of a third capture mesh 1313a. When retracted, the projecting struts 1313b can cut large thrombus, which are then collected in the interior of the third capture mesh 1313a. This design combines the advantages of the various types of capture units 131 and provides improved thrombectomy performance.

Referring to Figs. 15a to 15c, adjacent capture units 131 are connected by straight loading sections 131a. The proximal end of the most distal capture units (i.e., the first capture unit 1311) is attached to the supporting member 110 to form a distal joint, and the proximal end of the most proximal capture units (a second capture unit 1312 or a third capture unit 1313) is attached to the supporting member 110 to form a proximal joint. In the mesh capture device, the straight loading sections 131a located between the proximal and distal joints are not attached to the supporting member 110 to facilitate smooth blood flow. In the illustrated implementation, the proximal end of the most proximal second capture unit 1312 or third capture unit 1313 is fixed through a radiopaque ring 104, and the proximal end of the most distal first capture unit 131 is fixed through another radiopaque ring 104.

### <Embodiment 7>

Different from the foregoing embodiments, a medical device 100 according to a seventh embodiment of the present invention is used to remove a stone from a bile duct or ureter. The removal is accomplished in the substantially same manner as described above for thrombectomy.

In this embodiment, when a capture unit 131 is expanded, its capture mesh will come into contact with an inner wall of a lumen around a stone, while being movable within the lumen during a surgical procedure to extract the tone from duct and collect the stone in the capture mesh. Dense mesh openings in the capture mesh can effectively block the passage of small stone therethrough. In addition, a plurality of such capture units 131 may be coaxially arranged to form a stone removal structure with multiple layers, which can take repeated removal actions within the lumen, avoid any stone from remaining in the lumen and resulting in improved stone removal efficiency. Further, in the stone removal process, an external aspiration device may be activated to suck distal small stone into a catheter, increasing complete stone removal success.

As described above, the medical device and system of the present invention offer at least the following benefits:
Firstly, the distal opening of each of the first and second capture units is outwardly curved to from an umbrella-shaped surface. This structure has a small contact area with a lumen, which can reduce damage to a wall of the lumen during removal of a thrombus, stone or other occlusive object from the lumen. In addition, it can be retracted to better collect the occlusive object without falling off or escape of the occlusive object. Further, the capture units can be better guided back into the delivery device.

Secondly, the distal opening of the most distal first capture unit is completely outwardly curved and it is mainly intended to trap disrupted occlusive object, while the distal opening of each of the second capture units is partly outwardly curved. This structural design increases the compliance and adaptation within tortuous blood vessels and blood vessels with a varying diameter and thus lowers the likelihood of the device collapsing within such blood vessels. In addition, it reduces resistance to retraction into a catheter, facilitating delivery and withdrawal.

Thirdly, after coming into close contact with an inner wall of a lumen, the capture mesh of each capture unit may be retracted to effectively removal an occlusive object adhering to the wall of the lumen. This can achieve even better therapeutic outcomes, in particular for cases of old and strongly adherent occlusive objects. The curved umbrella-like capture meshes can collect one or more occlusive objects therein. In particular, more occlusive objects can be retained in the spaces between adjacent capture units, enabling more efficient removal of occlusive objects. Additionally, the capture units may present a mesh opening density gradually decreasing in a distal to proximal direction. With this arrangement, the denser mesh openings at the distal end enable collection of an occlusive object with a reduced risk of distal escape, and the sparser mesh openings at the proximal end allows large occlusive object to be disrupted during retraction of the medical device while the occlusive object are encapsulated and transported toward the aspiration tube, resulting in good removal performance and high removal efficiency.

Further, it will be recognized that while the invention has been described above with reference to preferred embodiments thereof, it is not intended to be limited to these embodiments. In light of the above teachings, any person familiar with the art may make many possible modifications and variations to the disclosed embodiments or adapt them into equivalent embodiments, without departing from the scope of the invention. Accordingly, it is intended that any and all simple variations, equivalent changes and modifications made to the foregoing embodiments based on the substantive disclosure of the invention without departing from the scope thereof fall within this scope.

## Claims

1. A medical device used to remove an occlusive object from a target lumen, comprising:
a supporting member; and
a mesh capture device mounted over an outer circumference of a distal end of the supporting member,
wherein the mesh capture device comprises a plurality of capture units that are sequentially arranged at an interval along an axial direction of the supporting member, wherein the plurality of capture units include a first capture unit and a plurality of second capture units, wherein the first capture unit is farther away from a proximal end of the supporting member than the second capture unit, wherein the first capture unit comprises a first capture mesh outwardly folded along an entire circumference thereof and curved proximally, wherein the second capture unit comprises a second capture mesh radially projecting outwardly along a part of a circumference thereof, wherein the second capture meshes of adjacent second capture units form a capture mesh that is along an entire circumference of the supporting member.

2. The medical device according to claim 1, wherein the second capture mesh is formed by a mesh tube that is outwardly folded along a part of a circumference thereof and is curved proximally; or by a mesh tube that radially projects outwardly along a part of a circumference thereof.

3. The medical device according to claim 1 or 2, wherein the plurality of capture units further include a third capture unit, wherein the first capture unit is farther away from the proximal end of the supporting member than the third capture unit, and wherein the third capture unit comprises a third capture mesh radially projecting outwardly along an entire circumference thereof.

4. The medical device according to claim 3, wherein the third capture mesh is formed by a mesh tube that radial projects outwardly along an entire circumference thereof and/or the third capture mesh is deflected proximally or distally.

5. The medical device according to claim 1 or 2, wherein: the plurality of capture units are integrally connected to form a single structure; or the plurality of capture units are disconnected to form separate structures.

6. The medical device according to claim 5, wherein in case of the plurality of capture units being integrally connected to form a single structure, the mesh capture device is formed from a single mesh tube; adjacent capture units are connected by a straight loading section; a most distal capture unit is proximally attached to the supporting member to form a distal joint; a most proximal capture units is proximally attached to the supporting member to form a proximal joint; and the straight loading section of the mesh capture device is located between the proximal and distal joints and is not attached to the supporting member.

7. The medical device according to claim 5, wherein in case of the plurality of capture units being disconnected to form separate structures, the mesh capture device is formed from a plurality of mesh tubes, wherein each capture unit is fabricated from a corresponding mesh tube and comprises a straight loading section, wherein the straight loading section is fixedly sleeved over the outer circumference of the distal end of the supporting member.

8. The medical device according to claim 2, wherein the second capture meshes is formed by the mesh tube that is outwardly folded along a part of the circumference thereof and is curved proximally, wherein each of the first capture mesh and/or the second capture meshes comprises a leading end and a trailing end opposite to each other along an axis thereof, wherein the leading end is proximally curved, wherein the trailing end is curved toward the leading end, and wherein a mesh opening density of the trailing end is lower than a mesh opening density of the leading end.

9. The medical device according to claim 2, wherein the second capture mesh is formed by the mesh tube that is outwardly folded along a part of the circumference thereof and is curved proximally, wherein each of the first capture mesh and/or the second capture meshes is outwardly curved at a trailing end thereof to form a non-invasive end, and wherein the non-invasive end is covered with a soft material.

10. The medical device according to claim 2, wherein the second capture mesh is formed by the mesh tube that radially projects outwardly along a part of the circumference thereof, and wherein the second capture mesh is defined by a portion of at least one shape selected from a sphere, a disk, a cylinder, a bag and a basket.

11. The medical device according to claim 3, wherein the third capture mesh is defined by at least one shape selected from a sphere, a disk, a cylinder, a bag and a basket.

12. The medical device according to claim 1 or 2, further comprising at least one of:
a radial dimension of at least one capture unit after fully expanded being greater than or equal to a diameter of the target lumen;
a mesh opening density of the capture mesh of at least some of the capture units gradually decreasing from center to periphery;
a mesh opening density of the mesh capture device gradually decreasing in a distal to proximal direction;
a radial dimension of the mesh capture device gradually increasing or decreasing in a proximal to distal direction; and
a graft provided on the first capture mesh, wherein the graft covers some of mesh openings of the first capture mesh.

13. The medical device according to claim 1 or 2, wherein the supporting member comprises a hollow core and a distal guide head, wherein the distal guide head is provided at a distal end of the hollow core, wherein the hollow core comprises a stiff section and an elastic section arranged along an axial direction from a proximal end to a distal end, and wherein the mesh capture device is disposed on the elastic section.

14. A medical system, comprising:
a medical device as defined in any one of claims 1 to 13;
a delivery device for loading and delivering the medical device into a target lumen; and
an aspiration device configured for connection with the delivery device and for application of a suction force through the delivery device for sucking an occlusive object inside the target lumen.
